# EUROPÄISCHE PATENTSCHRIFT

(11) **EP 0 444 382 B1**
(45) Veröffentlichungstag und Bekanntmachung des Hinweises auf die Patenterteilung: **06.04.1994**
(21) Anmeldenummer: 90810951.5
(22) Anmeldetag: 05.12.1990
(51) Int. Cl.: A61F 2/34

(54) **Zementfreie künstliche Hüftgelenkspfanne aus Metall**
Cementless metallic artificial hip cup
Prothèse d'acétabule métallique utilisable sans ciment

(30) Priorität: 01.03.1990 CH 643/90
(43) Veröffentlichungstag der Anmeldung: 04.09.1991
(73) Patentinhaber: SULZER Medizinaltechnik AG, 8404 Winterthur (CH); PROTEK AG, 3110 Münsingen-Bern (CH)
(72) Erfinder: Spotorno, Lorenzo, Prof.-Dr.-med., I-17024 Finale Ligure (IT); Koch,Rudolf, CH-8267 Berlingen (CH); Willi, Roland, CH-8543 Stadel (CH); Marchetti, Pier G., 4000 Bologna (IT); Luciano, Silvello,Prof., Dr., I-20021 Cassina Nuova, Bollate-Milano (IT)
(74) Vertreter: Hammer, Bruno, Dr.

(56) Entgegenhaltungen:
- EP-A- 0 066 092
- EP-A- 0 190 446
- EP-A- 0 253 941
- EP-A- 0 329 019
- GB-A- 2 069 338
- IT-A- 1 227 161
- LU-A- 77 518

## Beschreibung

Die Erfindung betrifft eine zementfrei implantierbare künstliche Hüftgelenkpfanne aus Metall, die aus einer Innenschale und aus einer Aussenschale besteht, welche einen Zwischenraum zwischen sich bilden wobei die Schalen an ihren Rändern fest und gasdicht miteinander verbunden sind, wobei im Zwischenraum ein elastisches Übertragungselement angeordnet ist, das sich auf beide Schalen abstützt, und wobei die Aussenschale dünnwandig und zum Zwischenraum hin nachgiebig ausgefüllt ist. Eine solche künstliche Hüftgelenkspfanne, die einen metallischen Hohlkörper mit annähernd konstanter Wandstärke bildet und mit einem elastischen, volumeninvarianten Material gefüllt ist, wird in EP-A-0 190 446 gezeigt. Eine zweiteilige künstliche Hüftgelenkspfanne mit einer elastischen und/oder dämpfenden Pufferschicht zwischen den Schalen ist in EP-A-0 066 092 gezeigt.

Die zitierten Ausführungen haben eine beschränkte Dämpfungswirkung zwischen der inneren und der äusseren Halbschale, indem geringe Relativbewegungen zwischen dem inneren und dem äusseren Schalenkörper möglich sind. Hingegen führt die Krafteinleitung am Knochengewebe zwangsläufig zu Spannungsspitzen, die durch die relativ starre Geometrie der Aussenschale bedingt sind. Oertliche Ueberbeanspruchungen von eingewachsenem Knochengewebe und daraus folgende Ablösungen können bei einer formstabilen Aussenschale nicht ausgeschlossen werden. Ebenso führen Veränderungen der ursprünglichen Knochenstruktur, die eine lokale Veränderung der Elastizität zur Folge haben, zu einer Schwächung der Bindung zwischen Aussenschale und Knochengewebe.

Hier schafft die Erfindung Abhilfe. Sie löst die Aufgabe, innerhalb bestimmter Grenzen eine ähnliche lokale Steifigkeit gegenüber Druckkräften, wie sie das einwachsende Knochengewebe aufweist, an der Kontaktfläche der Aussenschale zum Knochengewebe zu erzeugen und diese mit einer guten Einwachsstruktur zu versehen.

Gemäss der Erfindung wird die Aufgabe dadurch gelöst, dass die Aussenschale mit einem mehrlagigen Metallgewebe bedeckt ist und dass sie mit dem Metallgewebe unter von aussen einwirkenden Kräften in vorgegebenen Grenzen zur Innenschale hin deformierbar ist, während die Innenschale steif und formbeständig ist und eine Wandstärke von mehr als 2 mm aufweist, dass der Zwischenraum als Hohlraum ausgeführt ist und mindestens ein Übertragungselement aus gewelltem und/oder gestanztem Metallblech aufweist, und daß die Aussenschale und/oder das Metallgewebe am Schalenrand einen umlaufenden Wulst aufweisen, der die Primärverankerung am hinterschnittenen Knochengewebe unterstützt.

Die Vorteile der Erfindung sind darin zu sehen, dass zwischen der Aussenschale und dem einwachsenden Knochengewebe ein Kräfteausgleich ohne überhöhte Spannungsspitzen stattfindet, wenn Belastungswechsel auftreten. Ausserdem passt sich die äussere Halbschale längerfristigen Veränderungen der Lage des sie umgebenden Knochengewebes durch plastische Deformation an.

Der abhängige Patentanspruch 2 bezieht sich auf eine vorteilhafte Weiterbildung der Erfindung.

Im folgenden wird die Erfindung anhand von Ausführungsbeispielen beschrieben. Es zeigen:
- Fig. 1, 3, 5: einen Längsschnitt durch eine erfindungsgemässe Hüftgelenkspfanne mit einem einteiligen elastischen Uebertragungselement zwischen Innenschale und Aussenschale;
- Fig. 2: einen Längsschnitt durch eine erfindungsgemässe Hüftgelenkspfanne mit mehreren elastischen Uebertragungselementen zwischen Innenschale und Aussenschale;
- Fig. 4: einen Ausschnitt von Fig. 3 mit den Verbindungen an den Schalenrändern, und
- Fig. 6: den Ausschnitt einer Draufsicht auf ein einteiliges elastisches Uebertragungselement aus Fig. 5.

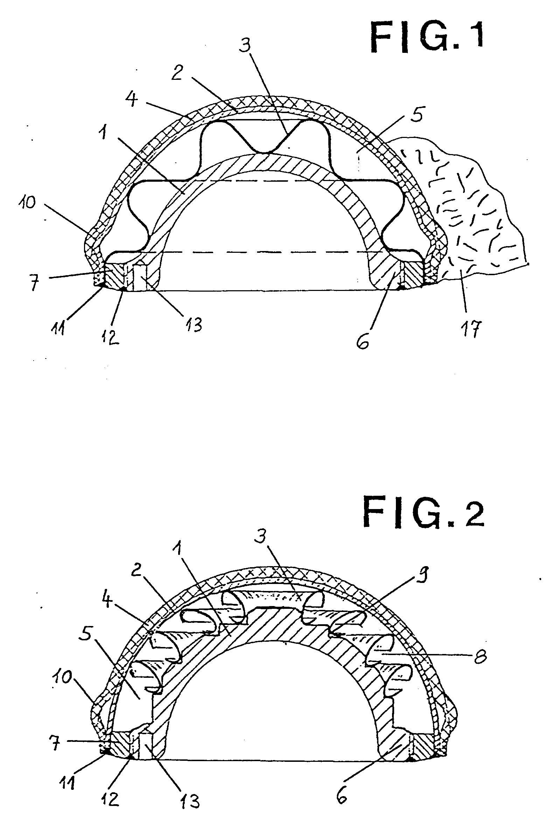

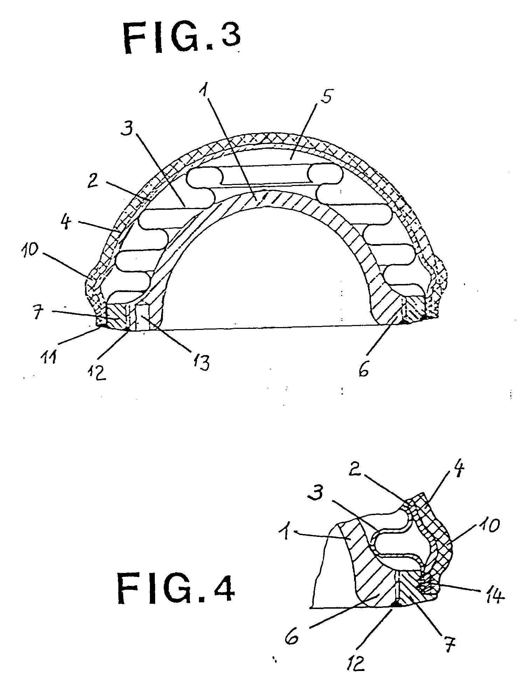

In den Fig. 1, 3 und 5 ist eine dünnwandige Aussenschale 2 auf ihrer Aussenseite mit einem mehrlagigen Metallgewebe 4 belegt. Wie auch in Fig. 4 gezeigt, sind am Schalenrand aussen beginnend Metallgewebe 4, Aussenschale 2, ein einteiliges elastisches Uebertragungselement 3 und ein Stützring 7 über eine Schweissung 11, 14 miteinander verbunden. Die Aussenschale und/oder das Metallgewebe 4 weisen einen umlaufenden, radial nach aussen weisenden Wulst 10 am Schalenrand auf, der nach innen federnd ausgeführt ist und der in einer passenden Hinterschneidung des Knochengewebes 17 beim Einsetzen der Hüftgelenksschale einrastet. Bei der Vormontage der beiden Schalen 1, 2 wird die Innenschale 1, die an ihrem äusseren Rand 6 ein Feingewinde aufweist, in einem passenden Innengewinde des Stützringes 7 eingeschraubt und nach Erreichen einer vorgeschriebenen Vorspannung des elastischen Uebertragungselementes 3 durch eine Schweissung 12 entlang des gemeinsamen Gewindes mit dem Stützring 7 verbunden. Das Drehmoment zum Einschrauben wird an Sacklöchern 13 der Innenschale 1 übertragen. Die richtige Verspannung des elastischen Uebertragsunselementes 3 zwischen den Schalen 1, 2 wird während des Einschraubens an verschiedenen Referenzpunkten der Aussenschale durch Messen der Steifigkeit in Richtung Schalenzentrum bestimmt.

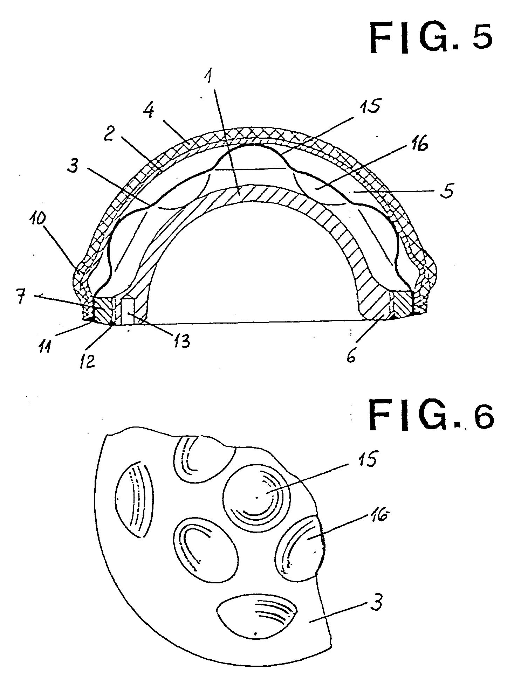

Der Hohlraum 5 zwischen Innenschale 1 und Aussenschale 2 kann auch mit mehreren elastischen Uebertragungselementen 3 versehen werden. So zeigt Fig. 2 eine Innenschale 1, die zum Hohlraum 5 Zentrierstufen 9 für torusähnliche einzelne elastische Uebertragungselemente 3, welche zwischen beiden Halbschalen 1, 2 verspannt sind, aufweist. Die einzelnen Uebertragungselemente 3 bestehen aus hohlen Metall-O-Ringen, die mit einem umlaufenden Schlitz 8 versehen sind. Die Federwirkung dieses O-Ringes 10 verändert sich zu einer sehr viel härteren Feder, sobald der Schlitz auf Null zusammengedrückt ist. Nichtlineare Federsysteme dieser Art haben den Vorteil, dass bei aussergewöhnlichen Belästungen nicht zu lange Federwege durchlaufen werden und dass die Formstabilität der ganzen Pfanne einigermassen erhalten bleibt. In Fig. 5 und 6 wird eine nichtlineare Federwirkung durch abplattbare Ausstülpungen 15, 16 eines elastischen Uebertragungselementes 3 erreicht, wobei Ausstülpungen 15 an der Aussenschale 2 und Ausstülpungen 16 an der Innenschale 1 anliegen.

Die Innenschale 1 bildet mit ihrer Steifigkeit eine Referenz für die elastischen Elemente der Pfanne und für den Kugelkopf des künstlichen Hüftgelenkes. Sie wird daher mit einer Wandstärke von mehr als 2 mm ausgeführt.

Aussenschale 2 und Innenschale 1 sind an ihren Rändern gasdicht miteinander verschweisst. Dies hat den Vorteil, dass nach dem Einsetzen der künstlichen Hüftgelenksschale weder mechanischer Abrieb noch sonstige Teilchen aus dem Hohlraum 5 an das umgebende Gewebe 17 gelangen.

## Patentansprüche

1. Zementfrei implantierbare künstliche Hüftgelenkpfanne aus Metall, die aus einer Innenschale (1) und aus einer Aussenschale (2) besteht, welche einen zwischenraum (5) zwischen sich bilden, wobei die Schalen (1, 2) an ihren Rändern fest und gasdicht miteinander verbunden sind, wobei im Zwischenraum (5) ein elastisches Übertragungselement (3) angeordnet ist, das sich auf beiden Schalen (1,2) abstützt, und wobei die Aussenschale (2) dünnwandig und zum Zwischenraum (5) hin nachgiebig ausgeführt ist, dadurch gekennzeichnet dass die Aussenschale mit einem mehrlagigen Metallgewebe (4) bedeckt ist und dass sie mit dem Metallgewebe unter von aussen einwirkenden Kräften in vorgegebenen Grenzen zur Innenschale (1) hin deformierbar ist, während die Innenschale (1) steif und formbeständig ist und eine Wandstärke von mehr als 2 mm aufweist, dass der Zwischenraum als Hohlraum ausgeführt ist und mindestens ein Übertragungselement (3) aus gewelltem und/oder gestanztem Metallblech aufweist, und daß dass die Aussenschale (2) und/oder das Metallgewebe (4) am Schalenrand einen umlaufenden Wulst (10) aufweisen, der die Primärverankerung am hinterschnittenen Knochengewebe (17) unterstützt.

2. Hüftgelenkpfanne nach Anspruche 1, dadurch gekennzeichnet, dass Übertragungselemente (3) aus Metallblech im Hohlraum (5) in Kombination mit der Aussenschale (2) gegenüber radial einwirkenden Aussenkräften als nichtlineares Federsystem ausgeführt sind.

## Claims

1. An artificial acetabulum made of metal, which can be implanted without cement, and which consists of an inner shell (1) and an outer shell (2), which between them form an intermediate space (5), whereby at their edges the shells (1, 2) are connected to one another in a secure and gas-tight manner, whereby in the intermediate member (5) is disposed an elastic transfer member (3), which is supported on both shells (1, 2), and whereby the outer shell (2) is constructed with thin walls and flexible towards the intermediate space (5),
**characterised in that** the outer shell is covered with a multi-layer metal gauze (4),
**and in that** with the metal gauze it can be deformed towards the inner shell (1) within 'prescribed limits under forces acting from outside, whereas the inner shell (1) is rigid and non-deformable and has a wall thickness of more than 2 mm,
**in that** the intermediate space is designed as a cavity and comprises at least one transfer member (3) made from corrugated and/or stamped metal sheet,
**and in that** the outer shell (2) and/or the metal gauze (4) on the shell edge comprise a circumferential bead (10), which supports the primary attachment at the undercut osseous tissue (17).

2. An acetabulum according to Claim 1,
**characterised in that** transfer members (3) made from metal sheet in the cavity (5) in combination with the outer shell (2) are designed as a non-linear spring system with respect to radially acting external forces.

## Revendications

1. Acétabule artificielle en métal, à implanter sans ciment, qui est constituée d'une coque intérieure (1) et d'une coque extérieure (2) formant entre elles un volume intermédiaire (5), les coques (1, 2) étant reliées entre elles sur leurs bords de manière fixe et étanche aux gaz, un élément de transmission (3) élastique, qui prend appui sur les deux coques (1, 2), étant prévu dans le volume intermédiaire (5) et la coque extérieure (2) présentant une paroi mince et étant réalisée de manière à céder vers le volume intermédiaire (5), caractérisée en ce que la coque extérieure est recouverte par un tissu métallique (4) à plusieurs couches, en ce qu'avec le tissu métallique elle est déformable vers la coque intérieure (1), dans des limites données, sous l'action de forces agissant de l'extérieur, tandis que la coque intérieure (1) est rigide et indéformable et présente une paroi d'une épaisseur supérieure à 2 mm, en ce que le volume intermédiaire est une cavité et comporte au moins un élément de transmission (3) en tôlé métallique ondulée et/ou emboutie et en ce que la coque extérieure (2) et/ou le tissu métallique (4) présente sur le bord de la coque un bourrelet (10) périphérique qui soutient l'ancrage primaire sur le tissu osseux (17) contre-dépouillé.

2. Acétabule selon la revendication 1, caractérisée en ce que les éléments de transmission (3) en tôle métallique dans la cavité (5) sont réalisés en combinaison avec la coque extérieure (2) par rapport à des forces extérieures agissant radialement, comme un système élastique non linéaire.
